(19) Europäisches Patentamt
European Patent Office
Office européen des brevets

(11) Publication number: **0 342 726 A1**

(12) **EUROPEAN PATENT APPLICATION**

(21) Application number: **89201015.8**

(51) Int. Cl.[4]: **C07D 271/06 , A61K 31/41**

(22) Date of filing: **20.04.89**

(30) Priority: **22.04.88 NL 8801048**

(43) Date of publication of application:
**23.11.89 Bulletin 89/47**

(84) Designated Contracting States:
**AT BE CH DE ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **Hulkenberg, Antonius**
**c/o OCTROOIBUREAU ZOAN B.V. P.O. Box 140**
**NL-1380 AC Weesp(NL)**
Inventor: **van der Heyden, Johannes A. M.**
**c/o OCTROOIBUREAU ZOAN B.V. P.O. Box 140**
**NL-1380 AC Weesp(NL)**
Inventor: **van Wijngaarden, Ineke**
**c/o OCTROOIBUREAU ZOAN B.V. P.O. Box 140**
**NL-1380 AC Weesp(NL)**

(74) Representative: **Muis, Maarten et al**
**OCTROOIBUREAU ZOAN B.V. P.O. Box 140**
**NL-1380 AC Weesp(NL)**

(54) **New 3,5-substituted 1,2,4-oxadiazoles.**

(57) The invention relates to a group of new 3,5-substituted 1,2,4-oxadizoles, and the prodrugs and salts thereof. The compounds are represented by the formula

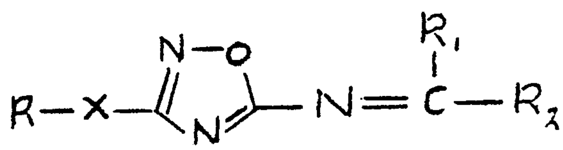

(2)

wherein
-R is a phenyl group which may be substituted with 1-3 halogen atoms, alkyl groups or alkoxy groups (1-4 C), or with $CF_3$, a group $-O-(CH_2)_m-O-$ wherein m has the value 1 or 2;
-X is the group $(-CH=CH-)_n$, wherein n is 0 or 1;
-$R_1$ is hydrogen or alkyl having 1-4 C-atoms, and
-$R_2$ is a group $-NR_3R_4$, wherein $R_3$ and $R_4$ are saturated or unsaturated alkyl having 1-4 C-atoms;
and the prodrugs and salts thereof with pharmacologically acceptable acids.
It has been found that these compounds have interesting anti-convulsive activities, and are less toxic in comparison with related known compounds.

EP 0 342 726 A1

## New 3,5-substituted 1,2,4-oxadiazoles.

The invention relates to a group of new 3,5-substituted 1,2,4-oxadiazoles having anti-convulsive properties, to the preparation of these compounds, and to pharmaceutical compositions comprising at least one of these compounds or a salt thereof as an active substance.

It is known from J. Med. Chem. 31 (1988), 7-11 that amidine derivatives of 1,3,4-thiadiazoles of formula 1

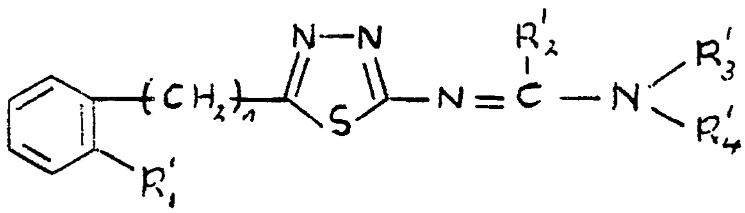

(1)

wherein n has the value 0 or 1, $R_1'$ is hydrogen, methyl, trifluoromethyl or phenyl, $R_2'$ is hydrogen, alkyl having 1-3 C-atoms, or benzyl, $R_3'$ is hydrogen, alkyl having 1-4 C-atoms or benzyl, $R_4'$ is hydrogen or ethyl, or wherein $R_3'$ and $R_4'$ together with the nitrogen atom, for example, represent the morpholino group, have an anticonvulsive activity. However, these compounds suffer from the drawback of being rather toxic.

It has been found surprisingly that compounds of formula 2

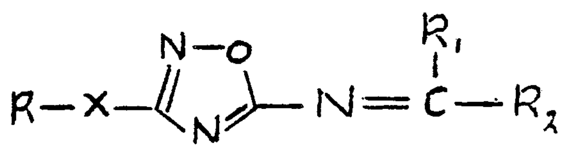

(2)

wherein

-R is a phenyl group which may be substituted with 1-3 halogen atoms, alkyl groups or alkoxy groups (1-4 C), or with $CF_3$, a group $-O-(CH_2)_m-O-$ wherein m has the value 1 or 2;

-X is the group $(-CH=CH-)_n$, wherein n is 0 or 1;

$-R_1$ is hydrogen or alkyl having 1-4 C-atoms, and

$-R_2$ is a group $-NR_3R_4$, wherein $R_3$ and $R_4$ are saturated or unsaturated alkyl having 1-4 C-atoms;

and the salts thereof with pharmaceutically acceptable acids have excellent anticonvulsive properties with a wide therapeutic index.

When a chiral centre is present, both the individual enantiomers and mixtures belong to the invention. If cis-trans isomers are possible both mixtures and the individual compounds are part of the invention.

The invention also includes the so-called prodrugs of the compounds of formula 2. Prodrugs are derivatives which as such are inactive and which after administration are converted in the body into an active compound of formula 2.

Suitable acids with which the compounds of formula 2 according to the invention may form pharmaceutically acceptable acid addition salts are, for example, inorganic acids, for example, hydrochloric acid, sulphuric acid, phosphoric acid, nitric acid, and organic acids, for example, citric acid, fumaric acid, maleic acid, tartaric acid, acetic acid, benzoic acid, p-toluenesulphonic acid, methane sulphonic acid, and the like.

The anticonvulsive activity of the compounds according to the invention was determined by means of a test in which a supramaximum quantity of pentamethylene tetrazole causing convulsions was administered intraveously in mice. The extent to which the compounds according to the invention prevent these convulsions is hence a measure of the anticonvulsive activity.

The compounds according to the invention and their salts can be brought into forms suitable for administra tion, for example, pills, tablets, coated tablets, capsules, powders, injection liquids and the like in the conventional manner and while using suitable auxiliary substances and carrier materials.

The dosage in which the active compounds according to the invention may be used depend on the severity and the nature of the disease to be treated and on the mode of administration.

The new compounds of formula 2 according to the invention can be prepared in a manner known for

analogous compounds. A suitable mode of preparation for this type of compounds is, for example, described in Chem. Pharm. Bull. 25, (1977), 3360 and in J. Het. Chem 8, (1971), 137.

The invention will now be described in greater detail with reference to the ensuing specific example.

## EXAMPLE I

1.5 ml of N,N-dimethylformamide dimethylacetal are added to a solution of 1.6 g of 3-phenyl-5-amino-1,2,4-oxadiazole in 8 ml of methanol. The reaction mixture is then refluxed for 3 hours. After cooling, the compound N,N-dimethyl-N′-(3-phenyl-1,2,4-oxadiazol-5-yl)formamidine (compound no. 1 of the table below) crystallises. After sucking off the crystals and washing with cold methanol, 1.6 g of this compound having a melting-point of 109.5° C are obtained.

The compounds 2 to 15 of the table below are obtained in an analogous manner. The compounds have the general formula

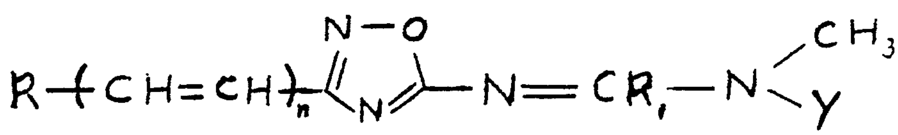

TABLE

| No. | n | R | $R_1$ | Y | Melt. p. °C |
|---|---|---|---|---|---|
| 1 | 0 | phenyl | H | $CH_3$ | 109.5 |
| 2 | 0 | 2,6-dichlorophenyl | H | $CH_3$ | 98 |
| 3 | 0 | 4-methoxyphenyl | H | $CH_3$ | 133 |
| 4 | 0 | 2-trifluoromethylphenyl | H | $CH_3$ | oil |
| 5 | 0 | phenyl | $CH_3$ | $CH_3$ | 104 |
| 6 | 0 | 2-methylphenyl | H | $CH_3$ | 63 |
| 7 | 0 | 2-trifluoromethylphenyl | $CH_3$ | $CH_3$ | 92 |
| 8 | 0 | 2-fluorophenyl | H | $CH_3$ | 106 |
| 9 | 0 | 2,4-difluorophenyl | H | $CH_3$ | 139 |
| 10 | 0 | 2,3-difluorophenyl | H | $CH_3$ | 119 |
| 11 | 0 | 2-methoxyphenyl | H | $CH_3$ | 109 |
| 12 | 0 | phenyl | H | $CH_2$-C≡CH | 109 |
| 13 | 1 | phenyl | H | $CH_3$ | 117 |
| 14 | 0 | 2,6-difluorophenyl | H | $CH_3$ | 108 |
| 15 | 0 | 4-fluorophenyl | H | $CH_3$ | 147 |

## EXAMPLE II

N-propargyl N-methyl N′-(3-phenyl-1,2,4-oxadiazole-5-yl)formamidine

To a solution of 1.5 g of 5-ethoxy formylamino-3-phenyl-1,2,4-oxadiazole in 50 ml of ether is added 1 ml of methylpropargylamine. After stirring for four hours at room temperature the reaction mixture is evaporated in vacuo and the residue is triturated with diisopropylether. The resulting precipitate is filtered off. In this manner 1.3 g of the desired product is obtained, having a melting point of 109° C.

**Claims**

1. Compounds of formula 2

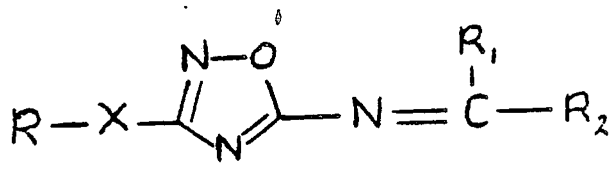

(2)

wherein
-R is a phenyl group which may be substituted with 1-3 halogen atoms, alkyl groups or alkoxy groups (1-4 C), or with $CF_3$, a group $-O-(CH_2)_m-O-$, wherein m has the value 1 or 2;
-X is the group $(-CH=CH-)_n$, wherein n is 0 or 1;
$-R_1$ is hydrogen or alkyl having 1-4 C-atoms, and
$-R_2$ is a group $-NR_3R_4$, wherein $R_3$ and $R_4$ are saturated or unsaturated alkyl having 1-4 C-atoms;
and the prodrugs and salts thereof with pharmacologically acceptable acids.

2. Pharmaceutical compositions which comprise at least one compound as claimed in Claim 1 as an active substance.

3. A method of preparing pharmaceutical compositions in which an active substance in brought into a form suitable for administration characterized in that a compound as claimed in Claim 1 is used as an active substance.

4. A method of preparing 3,5-substituted-1,2,4-oxadiazoles, characterized in that compounds as claimed in Claim 1 are prepared in a manner known for the synthesis of analogous compounds.

European Patent Office

# EUROPEAN SEARCH REPORT

Application Number

EP 89 20 1015

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | JOURNAL OF MEDICINAL CHEMISTRY, vol. 31, no. 1, January 1988, pages 7-11, American Chemical Society, Washington, DC, US; C.B. CHAPLEO et al.: "Substituted 1,3,4-thiadiazoles with anticonvulsant activity. 4. Amidines<br>* Whole article * | 1-4 | C 07 D 271/06<br>A 61 K 31/41 |
| Y | FR-A-2 100 787 (BOEHRINGER MANNHEIM)<br>* Whole document * | 1-4 | |
| Y | DE-A-2 240 887 (J.M.D. ARON-SAMUEL)<br>* Whole document * | 1-4 | |
| | | | TECHNICAL FIELDS SEARCHED (Int. Cl.4) |
| | | | C 07 D 271/00 |

The present search report has been drawn up for all claims

| Place of search | Date of completion of the search | Examiner |
|---|---|---|
| THE HAGUE | 10-07-1989 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document